## Europäisches Patentamt

## European Patent Office

## Office européen des brevets

(19)

(11) Veröffentlichungsnummer: **0 085 182**
**B1**

(12) **EUROPÄISCHE PATENTSCHRIFT**

(45) Veröffentlichungstag der Patentschrift:
23.04.86

(51) Int. Cl.⁴: **C 07 D 215/48,** C 07 D 215/50,
C 07 D 215/54

(21) Anmeldenummer: **82111949.2**

(22) Anmeldetag: **23.12.82**

(54) Verfahren zur Herstellung von Chinolinmonocarbonsäuren.

(30) Priorität: **28.01.82 DE 3202736**

(43) Veröffentlichungstag der Anmeldung:
**10.08.83 Patentblatt 83/32**

(45) Bekanntmachung des Hinweises auf die Patenterteilung:
**23.04.86 Patentblatt 86/17**

(84) Benannte Vertragsstaaten:
**BE CH DE FR GB IT LI NL**

(56) Entgegenhaltungen:
DE - A - 1 133 714
DE - A - 1 418 053
DE - A - 2 025 571

JOURNAL OF THE CHEMICAL SOCIETY, 1949, Seiten 2656-2662, London, GB., A.M. SPIVEY et al.: "Quinaldine and 4-hydroxyquinaldine derivatives from m-chloroaniline and m-toluidine"

Die Akte enthält technische Angaben, die nach dem Eingang der Anmeldung eingereicht wurden und die nicht in dieser Patentschrift enthalten sind.

(73) Patentinhaber: **BASF Aktiengesellschaft,**
**Carl-Bosch-Strasse 38, D-6700 Ludwigshafen (DE)**

(72) Erfinder: **Hagen, Helmut, Dr., Max-Slevogt-Strasse 17E,**
**D-6710 Frankenthal (DE)**
Erfinder: **Markert, Juergen, Dr., Am Speyerweg 26,**
**D-6704 Mutterstadt (DE)**

EP 0 085 182 B1

## Beschreibung

Die vorliegende Erfindung betrifft ein neues Verfahren zur Herstellung von Chinolinmonocarbonsäuren, ausgehend von Mono- oder Dihalogenmethylchinolinen oder Gemischen aus beiden.

Es ist bekannt, dass die Oxidation von Methylchinolinen mit Chromsäure zu den entsprechenden Carbonsäuren führt (Houben-Weyl 8/3, 392). Methylchinoline lassen sich auch über einen PdCl$_2$-Komplex mit Perhydrol zu Carbonsäuren oxidieren (Synthesis 1974, 819).

Ferner können an der Methylgruppe perhalogenierte Methylchinoline in saurem Milieu leicht zu Carbonsäuren verseift werden (Angew. Chem. 75, 235 (1963) ).

Vorteilhafter ist es, die Perhalogenierung zu vermeiden und die Mono- bzw. Dihalogenmethylchinoline in die Carbonsäure zu überführen. In alkalischen Medien gelingt dies durch Oxidation von Monohalogenmethylchinolinen mit Perhydrol (DE-OS 20 25 571).

Nach dem Vorschlag der DE-OS 14 18 053 stellt man Benzoesäuren durch Behandeln von Benzylchloriden mit Salpetersäure dar. Dieser Schritt der sauren, oxidativen Hydrolyse ist dagegen bei Mono- bzw. Dihalogenmethylchinolinen nicht bekannt, und diese Methode schien auch nicht anwendbar, da Verbindungen dieser Art als hydrolysebeständig gegen saure Medien beschrieben sind (Angew. Chem. 75, 235 (1969); Bull. Chem. Soc. Japan 29, 817 (1958) ).

Die DE-A 11 33 714 betrifft ein Verfahren zur Herstellung von ggf. chlorsubstituierten Chinolincarbonsäuren mit den Merkmalen des Oberbegriffs des Anspruchs 1. Die dort beschriebene Reaktion kann allerdings nur unter Druck durchgeführt werden, was nachteilig ist.

In J.Chem.Soc. 1949, Seiten 2659 und 2660 schliesslich ist die Verwendung von Schwefelsäure zur Hydrolyse von 5- bzw. 7-Chlor-ω-tribromchinaldin unter Bildung von 7-Chlorchinaldincarbonsäure beschrieben.

Es wurde nun überraschenderweise gefunden, dass man unsubstituierte oder durch ein oder zwei Halogenatome substituierte Chinolinmonocarbonsäuren, wobei die Carboxylgruppe und die Halogenatome jeweils die Positionen 2 bis 8 im Chinolinring einnehmen können, vorteilhaft erhält, wenn man unsubstituierte oder durch ein oder zwei Halogenatome substituierte Mono- oder Dihalogenmethylchinoline, in denen die Mono- oder Dihalogenmethylgruppe und die Halogenatome jeweils die Positionen 2 bis 8 im Chinolinring einnehmen können, in 60–80%iger Schwefelsäure mit 5–100%iger Salpetersäure in einem Temperaturbereich von 60 bis 140 °C behandelt.

Als Halogenverbindungen verwendet man zweckmässig jeweils die entsprechenden Brom- und Chlorverbindungen. Vorzugsweise setzt man dabei die Chlorverbindungen um. Man erhält sie z.B. durch Chlorierung der Methylchinoline nach Methoden, wie sie in Houben-Weyl 5/3, 725 f.

und 747 f. für stickstoffhaltige Heterocyclen beschrieben sind.

Im einzelnen wird das erfindungsgemässe Verfahren z.B. wie folgt durchgeführt:

Die reinen Mono- bzw. Dichlormethylchinoline oder vorzugsweise ihre Gemische, wie sie bei der Chlorierung anfallen, wobei beispielsweise auch Anteile von Trichlormethylchinolinen enthalten sein können, werden in 60 bis 80%iger, Schwefelsäure mit 5 bis 100%iger, vorzugsweise 30 bis 65%iger, Salpetersäure in einem Temperaturbereich von 60 bis 140 °C, vorzugsweise 100 bis 120 °C, für eine Dauer von 5 bis 50 Stunden, insbesondere 10 bis 30 Stunden, erhitzt.

Dabei legt man die Chlorierungsprodukte oder ihre Mischungen in der 3- bis 20-fachen, vorzugsweise 5- bis 10-fachen, Gewichtsmenge Schwefelsäure vor und gibt bei der entsprechenden Arbeitstemperatur die Salpetersäure in der 2- bis 10-fachen, vorzugsweise 3- bis 5-fachen, Molmenge, bezogen auf die Anzahl der Mole der vorhandenen Mono- bzw. Dichlormethylverbindungen, zu.

Der Vorteil dieses neuen Verfahrens liegt darin, dass in einer Eintopfreaktion Chlorierungsgemische, unter Vermeidung aufwendiger Trennungen in die Einzelkomponenten, in guter Ausbeute und hoher Reinheit zu Carbonsäuren umgesetzt werden.

Die Angaben in den Beispielen sind Gewichtsteile. Die Gemische sind gaschromatographisch bestimmt worden. Die Angaben bei den Säuren und Gemischen sind Gewichtsprozente.

Beispiel 1

3,7-Dichlor -8- chinolincarbonsäure

28 Teile 3,7-Dichlor -8- dichlormethylchinolin werden in 160 Teilen 70%iger Schwefelsäure auf 100 °C erhitzt. Innerhalb einer Stunde tropft man 20 Teile 65%iger Salpetersäure zu und rührt 10 Stunden bei 100 °C nach. Nach dem Abkühlen wird die Lösung mit 1000 Teilen Wasser versetzt, der ausgefallene Feststoff abgesaugt, mit Wasser neutral gewaschen und getrocknet. Man erhält 23 Teile (95% der Theorie) 3,7-Dichlor -8- chinolincarbonsäure, Fp. 278 °C.

Beispiel 2

3,7-Dichlor -8- chinolincarbonsäure

140 Teile Chlorierungsgemisch, bestehend aus 80% 3,7-Dichlor -8- dichlormethylchinolin und 20%, 3,7-Dichlor -8- monochlormethylchinolin, werden in 1500 Teilen 80%iger Schwefelsäure auf 100 °C erhitzt. Ab 60 °C wird mit der Zugabe von 250 Teilen 30%iger Salpetersäure begonnen, während dabei die Temperatur auf 100 °C gesteigert wird. Man rührt 20 Stunden bei 100 °C nach und arbeitet wie in Beispiel 1 auf. Es werden 106 Teile (87,5% der Theorie) 3,7-Dichlor -8- chinolincarbonsäure vom Fp. 278 °C erhalten.

Beispiel 3

3,7-Dichlor -8- chinolincarbonsäure

56 Teile Chlorierungsgemisch, bestehend aus 70% 3,7-Dichlor -8- monochlormethylchinolin, 24% 3,7-Dichlor -8- dichlormethylchinolin und 6% 3,7-Dichlor -8- trichlormethylchinolin werden in 300 Teilen 65%iger Schwefelsäure auf 100 °C erhitzt. Innerhalb von 3 Stunden tropft man 40 Teile 65%ige Salpetersäure zu und rührt anschliessend 12 Stunden bei 100 °C nach. Die Aufarbeitung erfolgt analog Beispiel 1. Dabei erhält man 95 Teile (93% der Theorie) 3,7-Dichlor -8- chinolincarbonsäure, Fp. 278 °C.

Analog Beispiel 1 bis 3 werden die Verbindungen 4 bis 8 hergestellt:

| | |
|---|---|
| 4) 3,5-Dichlor -8- chinolincarbonsäure | Fp. 131 °C |
| 5) 3,6-Dichlor -8- chinolincarbonsäure | Fp. 226 °C |
| 6) 3,8-Dichlor -6- chinolincarbonsäure | |
| 7) 6,8-Dichlor -2- chinolincarbonsäure | Fp. 189 °C |
| 8) 6-Chlor -8- chinolincarbonsäure | Fp. 225 °C |

**Beispiel 9**

6-Chlor -8- chinolincarbonsäure

26 Teile 6-Chlor -8- monobrommethylchinolin und 20 Teile 65%ige Salpetersäure werden unter Rühren in 150 Teilen 70%iger Schwefelsäure 6 Stunden auf 100 °C erwärmt. Danach wird abgekühlt und die Reaktionslösung mit 500 Teilen Wasser versetzt. Anschliessend wird mit Natronlauge neutralisiert und das dabei ausfallende Produkt abgesaugt, gewaschen und getrocknet. Durch Umkristallisieren aus N,N-Dimethylformamid erhält man 16 Teile (77% der Theorie) 6-Chlor -8- chinolincarbonsäure, Fp. 225 °C.

Analog Beispiel 9 werden die Verbindungen 10 bis 15 hergestellt:

| | |
|---|---|
| 10) 5-Chlor -8- chinolincarbonsäure | Fp. 210 °C |
| 11) 3,7-Dichlor -6- chinolincarbonsäure | Fp. 250 °C |
| 12) 7,8-Dichlor -2- chinolincarbonsäure | Fp. 202 °C |
| 13) 5,7-Dichlor -2- chinolincarbonsäure | Fp. 179 °C |
| 14) 7-Chlor -8- chinolincarbonsäure | Fp. 244 °C |
| 15) 7-Chlor -6- chinolincarbonsäure | Fp. 195 °C |

**Patentansprüche**

1. Verfahren zur Herstellung von unsubstituierten oder durch ein oder zwei Halogenatome substituierten Chinolinmonocarbonsäuren, wobei die Carboxylgruppe und die Halogenatome jeweils die Positionen 2 bis 8 im Chinolinring einnehmen können, unter Verwendung von Salpetersäure, dadurch gekennzeichnet, dass man unsubstituierte oder durch ein oder zwei Halogenatome substituierte Mono- oder Dihalogenmethyl-chinoline, in denen die Mono- oder Dihalogenmethylgruppe und die Halogenatome jeweils die Positionen 2 bis 8 im Chinolinring einnehmen können, in 60–80%iger Schwefelsäure mit 5–100%iger Salpetersäure (in einem Temperaturbereich von 60 bis 140 °C) behandelt.

2. Verfahren gemäss Anspruch 1, dadurch gekennzeichnet, dass man die Chinolinderivate in der Schwefelsäure mit einem 2- bis 10-fachen molaren Überschuss an Salpetersäure, bezogen auf die Mono- bzw. Dihalogenmethyl-chinoline, umsetzt.

3. Verfahren zur Herstellung der Chinolinmonocarbonsäuren gemäss Anspruch 1, dadurch gekennzeichnet, dass man ein Gemisch, bestehend aus den in Anspruch 1 genannten Mono- und Dihalogenmethylchinolinen in Schwefelsäure mit Salpetersäure behandelt.

4. Verfahren gemäss Ansprüchen 1 bis 3, dadurch gekennzeichnet, dass man als Halogenverbindungen jeweils die entsprechenden Chlorverbindungen verwendet.

**Revendications**

1. Procédé pour la préparation d'acides quinoléine-monocarboxyliques non substitués ou substitués par un ou deux atomes d'halogène, le groupe carboxyle et les atomes d'halogène pouvant occuper respectivement les positions 2 à 8 dans le noyau quinoléinique, avec utilisation d'acide nitrique, caractérisé en ce qu'on traite par de l'acide nitrique à 5–100%, dans de l'acide sulfurique à 60–80% et dans une gamme de température de 60 à 140 °C, des mono- ou di-halogénométhylquinoléines non substituées ou substituées par un ou deux atomes d'halogène, dans lesquelles le groupe mono- ou di-halogénométhyle et les atomes d'halogène peuvent occuper respectivement les positions 2 à 8 dans le noyau quinoléinique.

2. Procédé selon la revendication 1, caractérisé en ce qu'on fait réagir les dérivés de la quinoléine, dans l'acide sulfurique, avec un excès molaire de 2 à 10 fois d'acide nitrique par rapport aux mono- ou di-halogénométhylquinoléines.

3. Procédé pour la préparation des acides quinoléine-carboxyliques selon la revendication 1, caractérisé en ce qu'on traite par l'acide nitrique, dans l'acide sulfurique, un mélange composé des mono- et di-halogénométhylquinoléines définies dans la revendication 1.

4. Procédé selon l'une quelconque des revendications 1 à 3, caractérisé en ce qu'on utilise, en tant que composés halogénés, les composés chlorés respectivement correspondants.

**Claims**

1. A process for the preparation of a quinoline monocarboxylic acid which is unsubstituted or substituted by one or two halogen atoms, it being possible for the carboxyl group and the halogen atoms to each occupy one of positions 2 to 8 in the quinoline ring, using nitric acid, wherein a mono- or dihalomethyl quinoline which is unsubstituted or substituted by one or two halogen atoms and in which the mono- or dihalomethyl group and the halogen atoms may each occupy one of positions 2 to 8 in the quinoline ring, is treated in 60 to 80% strength sulphuric acid with 5 to 100% strength nitric acid at a temperature of from 60 to 140 °C.

2. A process as claimed in claim 1, wherein the quinoline derivative is reacted in the sulphuric acid with a 2- to 10-fold molar excess of nitric acid, based on the mono- or dihalomethyl quinoline.

3. A process for the preparation of a quinoline monocarboxylic acid as claimed in claim 1, wherein a mixture of the mono- and dihalomethyl quinolines mentioned in claim 1 is treated in sulphuric acid with nitric acid.

4. A process as claimed in claims 1 to 3, wherein the corresponding chlorine compound is used as halogen compound.